Europäisches Patentamt

European Patent Office

Office européen des brevets

(11) Publication number: **0 289 900**
**A1**

## EUROPEAN PATENT APPLICATION

(21) Application number: **88106593.2**

(51) Int. Cl.⁴: **A61K 47/00**

(22) Date of filing: **25.04.88**

(30) Priority: **30.04.87 US 44521**
**13.10.87 US 108175**

(43) Date of publication of application:
**09.11.88 Bulletin 88/45**

(84) Designated Contracting States:
**BE CH DE ES FR GB GR IT LI NL SE**

(71) Applicant: **ABBOTT LABORATORIES**

**Abbott Park Illinois 60064(US)**

(72) Inventor: **Hui, Ho-Wah**
**307 Albany Lane**
**Vernon Hills Illinois 60061(US)**
Inventor: **Vadnere, Madhu K.**
**344 Behm Drive**
**Grayslake Illinois 60030(US)**
Inventor: **Hsu, Chung-Chiang**
**26 Linden**
**Vernon Hills Illinois 60061(US)**

(74) Representative: **Modiano, Guido et al**
**MODIANO, JOSIF, PISANTY & STAUB**
**Modiano & Associati Baaderstrasse 3**
**D-8000 München 5(DE)**

(54) **Topical antibacterial compositions.**

(57) An antibacterial composition for topical administration, comprising from about 0.5 to about 10 percent of an antibacterial compound; from about one to about 30 percent of a non water soluble polymeric composition; from about .5 to about 40 percent of a plasticizer which plasticizes said polymeric composition; and from about 50 to about 95 percent of a solvent in which said polymeric composition and plasticizer are dissolved; whereby upon topical application of said antibacterial composition, said solvent will evaporate or penetrate the skin and leave a thin protective film of polymeric composition which retains said antibacterial compound against the skin.

# TOPICAL ANTIBACTERIAL COMPOSITIONS

This is a continuation-in-part of U.S. Serial No. 044,521 filed April 30, 1987.

This invention relates to topical antibacterial compositions.

There are many topical indications for antibacterial compounds. One important indication is acne. Many compositions including antibacterial compounds have been formulated for topical administration, but they have certain deficiencies. Some are water soluble so they are diluted by perspiration, or can be completely washed or worn off with water when the user swims or showers, for example. Thus, the water soluble compositions must be frequently applied. Other compositions do not penetrate very well into the skin, which is a requisite for some antibacterial compounds to be effective.

## Summary of the Invention

The topical antibacterial composition of this invention effectively penetrates the skin. At the same time, the composition resists washing and wear.

The antibacterial composition includes from about one to about 30 percent of a non-water soluble polymeric composition; from about 0.5 to about ten percent of an antibacterial compound; from about .5 to about forty percent of a plasticizer; and from about fifty to about ninety-five percent of a solvent in which said polymeric composition can be dissolved or dispersed. The polymeric composition retains the antibacterial compound on the skin, and forms a water and wear resistant film when the antibacterial composition is applied to the skin. The plasticizer prevents the film from cracking on the skin. Finally, the solvent allows the antibacterial composition to be easily applied, yet it evaporates and/or is absorbed into the skin, to leave a wear and water resistant film on the skin.

## Technical Disclosure

The antibacterial composition of the present invention is for topical application. It includes from about one to about 30 percent of a non water soluble polymeric composition, and from about 0.5 to about ten percent of an antibacterial compound. From about 0.5 to about forty percent of a plasticizer is added to the composition to plasticize the film and keep it flexible when it is applied to the skin. Finally, the antibacterial composition includes about 50 to 90 percent of a solvent which evaporates or penetrates the skin upon topical application to leave the plasticized polymeric film having the antibacterial compound in it against the skin. The resultant film resists water and wear, so frequent application of the formulation is not required.

Non water soluble polymeric compositions useful with this invention include ethylcellulose based polymers sold under the mark Aquacoat byFMC Corporation of Philadelphia, Pennsylvania; poly (methyl vinyl ether/maleic acid) polymers sold under the mark Gantrez by GAF Corporation; and copolymers of vinyl pyrrolidone and long chain -olefins sold under the mark Ganex by GAF. The Ganex polymer composition is preferred. These compositions are provided by the manufacturer containing certain solvents (e.g. alcohol) to make them workable in processing equipment. The solvent dries to leave a film when the polymer is applied to a surface on thin film. The polymers listed above are exemplary, other polymers compatible with the skin can also be used.

Antibacterial compounds which can be used in the formulation of this invention include erythromycin, tetracycline, clindamycin and meclocycline. Other bio-active agents (e.g., benzyl peroxide, retinoic acid, etc.) useful in treating skin conditions can also be included in the formulation. When antibacterial agents are used, however, the formulation can be used to treat infections on or immediately under the skin, including acne.

A volatile solvent is also part of the formulation. The amount of solvent to be added depends on the amount of solvent in the polymeric material provided by the manufacturer, usually the amount of volatile solvent already in the polymeric material is insufficient, so some solvent is added to bring the total solvent content to within the 50 to 90 percent range indicated. Virtually any non-toxic, non-irritating solvent can be added. It is preferred that the polymer be soluble in the added solvent so that the formulation can be spread on the skin to produce an even, uniform film, and to prevent the product from separating into layers. However, the polymer can be emulsified or dispersed in the solvent with emulsifiers, if necessary, so the polymer need not be soluble in the solvent. The solvent either should be volatile so that the polymer dries to form a film on the skin, or the solvent should be absorbable into the skin to leave a film on the skin.

A plasticizer is added to the composition to make the film, when the solvent dries or is absorbed, flexible so it will resist cracking. An oily substance compatible with the skin can be used.

However, some oily substances such as peppermint oil, eucalyptol oil, geranyl acetate or geraniol not only are compatible with skin and plasticize the film but enhance the penetration of antibacterial agents into the skin. For this reason, the above named oils are preferred in this invention. Because geranyl acetate and geraniol are sweet smelling, they are the most preferred. However, in addition to the above named plasticizers, tributylcifrate, diethyl phthalate and diethyl sebecate can be used as plasticizers.

Preparation and formulation of compositions of this invention will be described in the specific examples which follow:

Example 1
    2% erythromycin
    30% ethylcellulose
    1% sodium lauryl sulfate
    1% cetyl alcohol
    4% tributylcitrate (plasticizer)
    30% 190 proof ethanol
    32% distilled water

a) Ethylcellulose powder (30 gm) was dissolved in ethyl acetate (300 ml). Erythromycin (2 gm) was added to the mixture, and mixed thoroughly. The plasticizer tributylcitrate (4 grams) was added to the mixture, and mixed thoroughly.

b) An aqueous solution of sodium lauryl sulfate (1 gm) and cetyl alcohol (1 gm) was prepared in distilled water (32 ml).

c) The two mixtures from a and b above were combined by adding mixture b dropwise into mixture a, the surfactant (sodium lauryl sulfate) aiding in the emulsification of b into a.

d) The combined mixture was placed in a rotary evaporator to remove the ethyl acetate.

e) The ethyl alcohol (30 ml; 190 proof) was then added to the suspension of fine spheres of ethyl cellulose which formed during the procedure described in a - d above. A milky-white suspension of medicament was obtained.

When applied, the water and alcohol either evaporate of penetrate the skin to leave a plasticized film of ethylcellulose. The water/alcohol mixture form the "solvent" phase indicated above.

Example II
    21.3% micronized ethylcellulose
    0.7% cetyl alcohol
    0.8% sodium laurel sulfate

    2.0% erythromycin
    4.0% diethylphthalate
    25% ethanol
    46.3% distilled water

a) Aquacoat contains micronized ethylcellulose (30%), cetyl alcohol (1%), sodium laurel sulfate (1%) and 68% water. The aquacoat suspension (71 ml) was mixed with diethylphthalate (4 ml), a plasticizer.

b) Erythromycin base (2.0 gm) was dissolved in 190 proof ethanol (25 ml). The mixture from part a was then added, and mixed well. A sufficient quantity of water was added to reach a final volume of 100 ml for the total mixture. A milky white suspension was produced.

Example III
    2.0% (m/v) erythromycin base
    0.5% diethyl sebacate
    93.5% ethanol
    1.0% ethanol
    1.0% 2-amino-2 methyl propanol (AMP)
    5.0% ethyl monoesters of poly (methyl vinyl ether/maleic acid) polymer

a) Gantrez ES 225 is a commercially available solution which contains 50% ethyl monoesters of poly (methyl vinyl ether/maleic acid) polymer and 50% ethanol. It is sold by GAF Corporation of Wayne, New Jersey. Gantrez ES 225 (10 ml) was mixed with ethanol (88.5 ml). Erythromycin base ( 2 gm) was then added.

b) AMP (1.0 ml) and diethyl sebacate (.5 ml) were then added and mixed well with the mixture obtained from part a. AMP was added to neutralize the pH to about 7.0. Diethyl sebacate is a plasticizer. The resultant solution was clear.

Example IV
    2.0% (m/v) erythromycin base
    5.5% polyvinyl pyrrolidone/hexadecene copolymer
    94.0% isopropanol
    .5% diethyl sebacate

Ganex V-516 is a commercially available resin containing 55% polyvinyl pyrrolidone/hexadecene copolymer and 45% isopropanol. It is sold by GAF Corporation of Wayne, New Jersey. Ganex V-516 (10 ml) was mixed with 89.5 ml isopropanol. Diethyl sebacate (.5 ml) was added, and mixed well. Erythromycin base (2 gm) was then added and mixed until a clear solution was obtained.

## Example V

    4.0% (m/v) erythromycin base
        5.5% polyvinyl pyrrolidone/hexadecene copolymer
    94.0% isopropanol
    .5% diethylsebacate

The above formulation was prepared in the same fashion as in Exhibit IV above, except 4 grams of erythromycin base were added instead of 2 grams.

## Example VI

    2.0% (m/v) erythromycin base
    10.0% geranyl acetate
    84.5% isopropanol
        5.5% polyvinyl pyrrolidone/hexadecene copolymer

Erythromycin (2 gm) was added to isopropanol (80 ml) and geranyl acetate (10 ml), and mixed well until a clear solution was formed. Ganex V-516 (10 ml) was added, Ganex V-516 consisting of 55% polyvinyl pyrrolidone/mexadiene copolymer and 45% isopropanol. A clear solution resulted.

## Example VII

    2.0% (m/v) erythromycin base
    10.0% peppermint oil
    84.5% isopropanol
        5.5% polyvinyl pyrrolidone/hexadecene copolymer

The formulation was prepared the same way as described in Exhibit VI, except peppermint oil was used instead of geranyl acetate.

## Examples VIII - XV

    2.0% (m/v) erythromycin base
    10.0% plasticizer
    84.5% isopropanol
        5.5% polyvinyl pyrrolidone/hexadecene copolymer

These formulations were prepared the same way as Example VI, except the following plasticizers were used instead of geranyl acetate:

    Geraniol          (Example 8)
    Trans-cinnamaldehyde      (Example 9)
    (-)-carvyl propionate      (Example 10)
    p-anisaldehyde      (Example 11)
    (-)-carvyl acetate      (Example 12)
    dl-methylacetate      (Example 13)
    (-)-methone      (Example 14)
    cinnamylalcohol      (Example 15)

## Example XVI

    2.0% (m/v) erythromycin base
    5.0% geranyl acetate
    89.5% isopropanol
        5.5% polyvinyl pyrrolidone/hexadecene copolymer

This formulation was prepared according to the procedure of Example VI, except a lesser amount (5%) of geranyl acetate and a greater amount of solvent (89.5%) were used.

## Example XVII

    2.0% (m/v) erythromycin base
    1.0% geranylacetate
    93.5% isopropanol
        5.5% polyvinyl pyrrolidone/hexadecene copolymer

This formulation was prepared according to the procedure of Example VI, except a lesser amount (1.0%) of geranyl acetate and a greater amount (93.5) of solvent were used.

## Example XVIII

    2.0% (m/v) erythromycin base
    10.0% geraniol
    10.0% ganex V-216
    80.0% 200 ethanol

Ganex V-216 is a commercially available resin containing 100% active copolymer of polyvinyl pyrrolidone and hexadecene. It is sold by GAF Corporation of Wayne, New Jersey. Erythromycin base (2 gm) was mixed with 80 ml 200 proof ethanol. 10 ml geraniol was then added and mixed well. Then 10 ml ganex V-216 was added and mixed well.

## Example XIX

    2.0% (m/v) erythromycin base
    5.0% geraniol
    10.0% ganex V-216
    85.0% 200 proof ethanol

Example XIX was prepared the same way as in Example XVIII, except different amounts of geraniol and ethanol were used.

Example XX

2.0% (m/v) erythromycin base
5.0% geraniol
5.0% ganex V-216
90.0% 200 proof ethanol

Example XX was prepared the same was as in Example XVIII, except different amounts of geraniol, ganex V-216 and ethanol were used.

Example XXI

2.0% (m/v) erythromycin base
10.0% geraniol
5.0% ganex V-216
85.0% 200 proof ethanol

Example XXI was prepared the same way as described in Example XVIII, except different amounts of ganex V-216 and ethanol were used.

Example XXII

2.0% (m/v) erythromycin base
2.0% geraniol
10.0% ganex V-216
88.0% 200 proof ethanol

Example XXII was prepared the same way as described in Example XVIII, except different amounts of geraniol and ethanol were used.

Example XXIII

2.0% (m/v) erythromycin base
10.0% geraniol
10.0% ganex V-216
80.0% isopropanol

The formulation was prepared the same way as described in Example XVIII, except that isopropanol was used instead of ethanol.

It will be understood that various changes and modifications can be made in the details of procedure, formulation and use without departing from the spirit of the invention, especially as defined in the following claims.

Claims

1. An antibacterial composition for topical administration, comprising:
a) from about 0.5 to about 10 percent of an antibacterial compound;
b) from about one to about 30 percent of a non water soluble polymeric composition;
c) from about .5 to about 40 percent of a plasticizer which plasticizes said polymeric composition; and
d) from about 50 to about 95 percent of a solvent in which said polymeric composition and plasticizer are dissolved;
whereby upon topical application of said antibacterial composition, said solvent will evaporate or penetrate the skin and leave a thin protective film of polymeric composition which retains said antibacterial compound against the skin.

2. The antibacterial composition as recited in claim 1 wherein said plasticizer is an oil which enhances the skin penetration of said antibacterial compound.

3. The antibacterial composition as recited in claim 2 wherein said plasticizer is selected from peppermint oil, eucalyptol oil, geranyl acetate or geraniol.

4. The antibacterial composition as recited in claim 3 which includes from about one to about ten percent plasticizer.

5. The antibacterial composition as recited in claim 1 in which said polymeric composition is selected from ethylcellulose, vinylpyrrolidone, poly-(methyl vinyl ether/maleic acid polymers or copolymers of polyvinyl pyrrolidone and hexadecene.

6. The antibacterial composition as recited in claim 1 wherein said antibacterial compound includes erythromycin.

7. An antibacterial composition for topical administration, comprising:
a) from about 0.5 to about 10 percent of an antibacterial compound;
b) from about one to about 30 percent of a non water soluble polymeric composition selected from ethylcellulose, vinylpyrrolidone, poly(methyl vinyl ether/maleic acid polymers or copolymers of polyvinyl pyrrolidone and hexadecene;
c) from about .5 to about 40 percent of a plasticizer which plasticizes said polymeric composition, said plasticizer selected from peppermint oil, eucalyptol oil, geranyl acetate or geraniol; and
d) from about 50 to about 95 percent of a solvent in which said polymeric composition and plasticizer are dissolved;
whereby upon topical application of said antibacterial composition, said solvent will evaporate or penetrate the skin and leave a thin protective film of polymeric composition which retains said antibacterial compound against the skin.

8. An antibacterial composition for topical administration, comprising:

a) from about one half to about 10 percent of erythromycin;

b) from about one to about 30 percent of a non water soluble polymeric composition selected from ethylcellulose, vinylpyrrolidone poly(methyl vinyl ether/maleic acid polymers or copolymers of polyvinyl pyrrolidone and hexadecene;

c) from about one to about 10 percent of a plasticizer which plasticizes said polymeric composition, said plasticizer selected from peppermint oil, eucalyptol oil, geranyl acetate or geraniol; and

d) from about 50 to about 95 percent of a solvent in which said polymeric composition and plasticizer are dissolved;

whereby upon topical application of said antibacterial composition, said solvent will evaporate or penetrate the skin and leave a thin protective film of polymeric composition which retains said antibacterial compound against the skin.

9. An antibacterial composition for topical administration, comprising:

a) from about 0.5 to about 10 percent of erythromycin;

b) from about one to about 30 percent of a copolymer of polyvinyl pyrrolidone and hexadecene;

c) from about one to about 10 percent of geraniol; and

d) from about 50 to about 95 percent of ethanol;

whereby upon topical application of said antibacterial composition, said ethanol will evaporate or penetrate the skin and leave a thin protective film of the copolymer which retains the erythromycin against the skin.

10. The antibacterial composition as recited in claim 9 wherein the composition comprises 2% (m/v) erythromycin base; 10% geraniol; 10% copolymer, and 80% 200 proof ethanol.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 047 874 (L. NOUVEL) * Page 1, lines 11-19; page 1, line 35 - page 3, line 35; claims 1-11 * | 1-10 | A 61 K 47/00 |
| Y | WO-A-8 605 391 (B. GLUCK) * Claims 1-8 * | 1-10 | |
| Y | CHEMICAL ABSTRACTS, vol. 100, no. 26, June 1984, page 345, no. 215535x, Columbus, Ohio, US; & JP-A-59 29 615 (TERUMO CORP.) 16-02-1984 * Abstract * | 1-10 | |
| A | EP-A-0 085 334 (PLOUGH INC.) * Claims 1-10 * | | |
| A,P | CHEMICAL ABSTRACTS, vol. 107, 24th August 1987, page 355, no. 64631s, Columbus, Ohio, US; R. OTERI et al.: "A new waterproofing agent for sunscreen products" & COSMET. TOILETRIES 1987, 102(3), 107-9 | | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 23-08-1988 | TZSCHOPPE,D.A. |

EPO FORM 1503 03.82 (P0401)